# EUROPÄISCHE PATENTANMELDUNG

(11) **EP 0 531 826 A2**
(43) Veröffentlichungstag der Anmeldung: **17.03.1993**
(21) Anmeldenummer: 92114712.0
(22) Anmeldetag: 28.08.1992
(51) Int. Cl.: C07C 51/60

(54) **Verfahren zur Herstellung von Carbonsäurehalogeniden**

(30) Priorität: 07.09.1991 DE 4129822
(71) Anmelder: BASF Aktiengesellschaft, D-67063 Ludwigshafen (DE)
(72) Erfinder: Wettling, Thomas, Dr., W-6703 Limburgerhof (DE); Henkelmann, Jochem, Dr., W-6700 Ludwigshafen (DE); Troetsch-Schaller, Irene, Dr., W-6710 Frankenthal (DE); Hupfer, Leopold, Dr., W-6701 Friedelsheim (DE); Franzischka, Wolfgang, Dr., W-6710 Frankenthal (DE); Koehler, Hermann, Dr., W-6719 Bobenheim (DE)

(57) **Zusammenfassung**

Verfahren zur Herstellung von Säurehalogeniden der allgemeinen Formel I 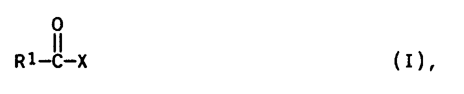
in der
- R¹: C₃- bis C₈-Cycloalkyl, C₄- bis C₂₀-Cycloalkyl-alkyl oder gegebenenfalls durch C₂- bis C₄-Acyl und/oder C₂- bis C₄-Acyloxy ein- bis dreifach substituiertes C₁- bis C₃₀-Alkyl, C₂- bis C₂₀-Alkenyl, C₂- bis C₂₀-Alkinyl oder gegebenenfalls durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein- bis dreifach substituiertes Aryl, C₇- bis C₂₀-Aralkyl, Heterocycloalkyl oder C₃- bis C₂₀-Heterocycloalkyl-alkyl und
- X: Chlor oder Brom
bedeuten, durch Umsetzung von Carbonsäuren der allgemeinen Formel II 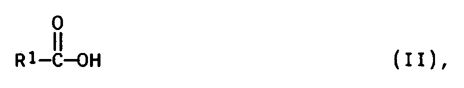
in der R¹ die oben genannten Bedeutungen hat, oder deren Anhydriden mit einem anorganischen Säurehalogenid der allgemeinen Formel III

O = AX₂ (III),

in der
- A: Kohlenstoff oder Schwefel
bedeutet und X die oben genannten Bedeutungen hat, indem man die Umsetzung bei Temperaturen von 40 bis 180°C in Gegenwart einer ungesättigten Stickstoffverbindung durchführt.

## Beschreibung

Die vorliegende Erfindung betrifft ein neues Verfahren zur Herstellung von Carbonsäurehalogeniden aus Carbonsäuren oder deren Anhydriden mit einem anorganischen Säurehalogenid in Gegenwart von ungesättigten Stickstoffverbindungen bei erhöhten Temperaturen.

Es ist bekannt, daß organische Carbonsäuren mit Phosphorpentachlorid, Phosphoroxichlorid, Phosphortrichlorid oder Thionylchlorid unter Bildung von Carbonsäurechloriden reagieren. Thionylchlorid ist unter den genannten Chloriden dadurch gekennzeichnet, daß es nur gasförmige Zersetzungsprodukte bildet, ist aber gleichzeitig das am wenigsten reaktionsfähige und wird daher im Überschuß eingesetzt (Organikum, 5. Auflage 1965, Seite 408, VEB Deutscher Verlag der Wissenschaften). Bei manchen Säurechloriden bereitet die spätere Abtrennung des überschüssigen Thionylchlorids jedoch Schwierigkeiten und stellt in jedem Falle einen zusätzlichen Reaktionsschritt dar, während andererseits bei Verwendung äquivalenter Mengen oder eines zu geringen Überschusses nicht umgesetzte Carbonsäure zurückbleibt und die Trennung ihrerseits erschwert. Die geringe Reaktivität ist weiterhin mit langen Reaktionszeiten und ungenügenden Ausbeuten verbunden.

Daher wurden reaktionsfördernde Zusätze empfohlen, wie Pyridin (J. Chem. Soc.(1953), 2117) oder Carbonsäureamide wie N,N-Dimethylformamid (Helv. Chim. Acta 42 (1959), 1654). Die genannten Beschleuniger haben jedoch entscheidende Nachteile, so z.B. die Cancerogenität des aus Dimethylformamid und Thionylchlorid entstehenden N,N-Dimethylcarbamoylchlorids (Chem. Abstr. Vol.75, 48 340 m; Chem. Abstr. Vol.77, 97 540b). Es ist weiterhin schwierig, geringste Reste dieser Beschleuniger aus dem Reaktionsprodukt zu entfernen, wie dies bereits für überschüssiges Thionylchlorid geschildert wurde. Für Phosgen, das ohne einen Beschleuniger im allgemeinen nur mangelhaft oder überhaupt nicht reagiert, gilt Entsprechendes. Reste solcher organischen Beschleuniger können die Weiterverwendung der Carbonsäurechloride stark beeinträchtigen.

Der vorliegenden Erfindung lag daher die Aufgabe zugrunde, den zuvor genannten Nachteilen abzuhelfen.

Demgemäß wurde ein neues und verbessertes Verfahren zur Herstellung von Säurehalogeniden der allgemeinen Formel I 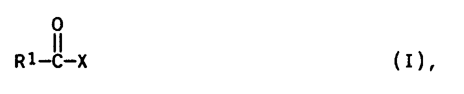
in der
- R¹: C₃- bis C₈-Cycloalkyl, C₄- bis C₂₀-Cycloalkyl-alkyl oder gegebenenfalls durch C₂- bis C₄-Acyl und/oder C₂- bis C₄-Acyloxy ein- bis dreifach substituiertes C₁- bis C₃₀-Alkyl, C₂- bis C₂₀-Alkenyl, C₂- bis C₂₀-Alkinyl oder gegebenenfalls durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein- bis dreifach substituiertes Aryl, C₇- bis C₂₀-Aralkyl, Heterocycloalkyl oder C₃- bis C₂₀-Heterocycloalkyl-alkyl und
- X: Chlor oder Brom
bedeuten, durch Umsetzung von Carbonsäuren der allgemeinen Formel II 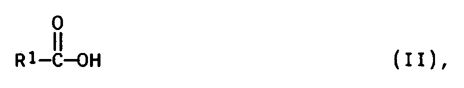
in der R¹ die oben genannten Bedeutungen hat, oder deren Anhydriden mit einem anorganischen Säurehalogenid der allgemeinen Formel III

O = AX₂ (III),

in der
- A: Kohlenstoff oder Schwefel
bedeutet und X die oben genannten Bedeutungen hat, welches dadurch gekennzeichnet ist, daß man die Umsetzung bei Temperaturen von 40 bis 180°C in Gegenwart einer ungesättigten Stickstoffverbindung durchführt.

Das erfindungsgemäße Verfahren kann wie folgt durchgeführt werden:
Die Carbonsäure II, deren Anhydride oder Mischungen aus diesen können in Gegenwart von ungesättigten Stickstoffverbindungen gegebenenfalls in Gegenwart eines inerten Lösungsmittels vorgelegt und auf erhöhte Temperaturen gebracht werden. Das entsprechende Säurehalogenid kann in die Reaktionsmischung getropft bzw. eingegast werden.

Die Umsetzung kann bei Temperaturen von 40 bis 180°C, bevorzugt von 70 bis 160°C, besonders bevorzugt von 80 bis 130°C und Drücken von 0,01 bis 50 bar, bevorzugt von 0,5 bis 5 bar, besonders bevorzugt bei Normaldruck (Atmosphärendruck) durchgeführt werden.

Als ungesättigte Stickstoffverbindungen (Katalysatoren, Reaktionsbeschleuniger) eignen sich insbesondere Guanidinderivate der allgemeinen Formel IV bzw. Isonitrile der allgemeinen Formel V in Mengen von 0,001 bis 10 Mol %, bevorzugt 0,01 bis 5 Mol %, besonders bevorzugt 0,1 bis 1 Mol % bezogen auf die eingesetze Carbonsäure II bzw. deren Anhydride.

Sofern die umzusetzende Carbonsäure II bzw. das Säurehalogenid I unter den Reaktionsbedingungen flüssig ist, kann ohne Lösungsmittel gearbeitet werden. Wenn es sich um hochschmelzende Verbindungen handelt, sollte im oberen Bereich der angegebenen Temperaturen gearbeitet werden. Für den Fall, daß das herzustellende Säurehalogenid unter den Reaktionsbedingungen flüssig ist, kann das Säurehalogenid, etwa aus einer früheren Charge, vorgelegt werden und als Ersatz für ein inertes Lösungsmittel als Reaktionsmedium dienen.

Als anorganische Säurehalogenide III kommen in Frage: Phosgen, Thiophosgen, Carbonyldibromid, Thionylchlorid und Thionylbromid, bevorzugt sind Phosgen und Thionylchlorid. Das anorganische Säurehalogenid III kann in Mengen von 1 bis 1,5 Mol, bevorzugt 1,1 bis 1,3 Mol, pro Mol Carbonsäure II bzw. deren Anhydride eingesetzt werden. Für den Fall, daß die umzusetzende Carbonsäure II mehr als eine Carboxylgruppe enthält, werden die genannten Zahlen mit der Zahl der Carboxylgruppen multipliziert.

Als inerte Lösungsmittel eignen sich beispielsweise aliphatische und aromatische Kohlenwasserstoffe wie Cyclohexan, Isododecan, Petrolether, Kerosin, Benzol, Toluol und die Xylole; aliphatische und aromatische Halogenkohlenwasserstoffe, wie Chloroform, Ethylidendichlorid, Ethantetrachlorid, Chlorbenzol und Dichlorbenzol.

Die im erfindungsgemäßen Verfahren herstellbaren Carbonsäurehalogenide können vom eingesetzten Katalysator abdestilliert werden oder zusammen mit diesem weiterverarbeitet werden. Durch Abdestillieren lassen sich die Katalysatoren in vielen Fällen mehrfach wiederverwenden, so daß der Verbrauch extrem gering wird.

Die Substituenten R¹, R², R³, R⁴, R⁵, R⁶, R⁷, X und A in den Verbindungen I, II, III, IV und V haben folgende Bedeutungen:
- R¹,R²,R³, R⁴,R⁵,R⁶ und R⁷: - C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- C₄- bis C₂₀-Cycloalkyl-alkyl, bevorzugt C₄- bis C₁₂-Cycloalkyl-alkyl wie Cyclopentyl-methyl, Cyclopropyl-methyl, Cyclohexyl-methyl und Cyclopentyl-ethyl,
- C₁- bis C₃₀-Alkyl, bevorzugt C₂- bis C₂₀-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₂- bis C₄-Alkyl wie Ethyl, n-Propyl, iso-Propyl, n-Butyl, iso-Butyl, sec.-Butyl und tert.-Butyl,
- C₂- bis C₂₀-Alkenyl, bevorzugt C₂- bis C₈-Alkenyl wie Vinyl, Allyl, But-2-en-1-yl, But-4-en-1-yl, But-4-en-2-yl, Pent-2-en-1-yl und 2,2-Dimethyl-pent-1-en-1-yl, besonders bevorzugt C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl und 5-Hexenyl,
- C₂-C₂₀-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl und 5-Hexinyl,
- durch C₂- bis C₇-Acyl und/oder C₂- bis C₇-Acyloxy ein- bis dreifach substituiertes C₁- bis C₃₀-Alkyl, bevorzugt Acetyl, Benzoyl, Acetyloxy und Benzoyloxy,
- durch C₂- bis C₄-Acyl und/oder C₂- bis C₄-Acyloxy ein- bis dreifach substituiertes C₂- bis C₂₀-Alkenyl, bevorzugt Vinylacetyl und Vinylacetyloxy,
- durch C₂- bis C₄-Acyl und/oder C₂- bis C₄-Acyloxy ein- bis dreifach substituiertes C₂- bis C₂₀-Alkinyl, bevorzugt Ethinacetyl und Ethinacetyloxy,
- Aryl wie Phenyl, 1-Naphthyl, 2-Naphthyl, 9-Naphthyl und Biphenyl , bevorzugt Phenyl,
- C₇- bis C₂₀-Aralkyl, bevorzugt C₇- bis C₁₂-Aralkyl wie Benzyl, Phenyl-ethyl, 1-Naphthyl-methyl und Biphenyl-methyl;
- Heterocycloalkyl, wie ein 5- oder 6-gliedriger Ring mit einem oder zwei O-, N- und/oder S-Atomen im Ring, der aromatisch oder nicht aromatisch sein kann, bevorzugt 2- oder 3-Furyl, 2- oder 3-Thienyl, 2- oder 3-Pyrrolyl, 2- oder 4-Imidazolyl, 2- oder 3-Oxazolyl, 2- oder 3-Thiazolyl, Pyridinyl, Morpholyl, Thiomorpholyl und Pyrazolyl,
- durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein-bis dreifach substituiertes Aryl, bevorzugt Nitrophenyl, Chlorphenyl, Dichlorphenyl, Cyanophenyl, Dicyanophenyl, Tolyl, Dimethylaminophenyl, Mesityl und Xylyl,
- durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein- bis dreifach substituiertes C₇- bis C₂₀-Aralkyl, bevorzugt Nitrobenzyl, Chlorbenzyl, Dichlorbenzyl, Methyltolyl, Methylmesityl und Methylxylyl,
- durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein-bis dreifach substituiertes Heterocycloalkyl, bevorzugt mono-Halogen, Nitro, Cyano oder Methyl, substituiertes Heterocycloalkyl wie 2-Methylimidazolyl, 1-Methylimidazolyl, 2-Methylpyridinyl, 3-Methylpyridinyl und 2-Methylpyrrolyl,
- durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein- bis dreifach substituiertes C₃- bis C₂₀-Heterocycloalkyl-alkyl,
- R²,R³,R⁴, R⁵ und R⁶: - zusätzlich Wasserstoff,
- X: - Chlor und Brom, bevorzugt Chlor,
- A: - Kohlenstoff oder Schwefel, bevorzugt Kohlenstoff.

Besonders bevorzugte Carbonsäuren II und Carbonsäurehalogenide I sind:

| Carbonsäure II | Carbonsäurehalogenid I |
|---|---|
| 2-Ethylhexansäure | 2- Ethylhexansäurechlorid |
| Cyclopropancarbonsäure | Cyclopropansäurechlorid |
| Pivalinsäure | Pivalinsäurechlorid |
| Talgfettsäure | Talgfettsäurechlorid |
| Neodecansäure | Neodecansäurechlorid |
| Isononansäure | Isononansäurechlorid |
| Carbonsäure | Carbonsäurechlorid |
| Behensäure | Behensäurechlorid |

Besonders bevorzugte ungesättigte Stickstoffverbindungen sind Guanidinderivate IV wie N,N,N',N'-Tetramethylguanidin, Pentamethylguanidin, N,N',N',N'',N''-Phenyl-tetramethylguanidin, 1,3-Diphenylguanidin.

Besonders bevorzugte ungesättigte Stickstoffverbindungen sind ferner Isonitrile V wie Cyclohexylisonitril, tert.-Butylisonitril, Isocyanessigsäuremethylester, 1,1,3,3-Tetramethylbutylisonitril.

Carbonsäurehalogenide sind bekannte Substanzen, die beispielsweise für Acylierungen eingesetzt werden können.

Sie finden beispielsweise Verwendung als Ausgangsstoffe für die Herstellung von Lösungsmitteln, Riechstoffen, Weichmachern, Waschmitteln, Peroxiden, Beizen und Pflanzenschutzmitteln.

### Beispiele

### Beispiel 1

In einem mit Thermometer, Rührer, Gaseinleitungsrohr und Rückflußkühler versehenen Kolben werden 144 g (1 mol) 2-Ethylhexansäure nach Zugabe von 1,1 g (0,01 mol, d.h. 1,0 mol % bezogen auf die Carbonsäure) Cyclohexylisonitril auf 120°C erwärmt und bei dieser Temperatur so lange Phosgen eingeleitet, bis kein Phosgen mehr verbraucht wird. In dem auf -78°C gehaltenen Kohlesäurekühler setzt dann starker Phosgenrücklauf ein und die Temperatur des Kolbeninhalts fällt leicht ab. Auf diese Weise werden innerhalb von 2 Stunden 120 g (1,2 Mol) Phosgen eingeleitet. Man läßt noch etwa 1 Stunde nachreagieren und entfernt dann den Tiefkühler, damit der größte Teil des noch in Lösung befindlichen Phosgens ausgasen kann. Den Rest entfernt man durch Ausblasen mit Stickstoff. Man erhält so 160,3 g (0,985 mol; 98,5 %) gelbes 2-Ethylhexansäurechlorid (Sdp. 67°C/11 Torr) das neben Spuren des Katalysators nur noch als Verunreinigung weniger als 0,5 % des entsprechenden Carbonsäureanhydrids enthält. Das Carbonsäurechlorid kann ohne weitere Reinigungsoperation weiterverarbeitet werden oder zum Zwecke der Katalysatorrückgewinnung und Produktreinigung z.B. destilliert werden.

### Beispiel 2

Nach der in Beispiel 1 beschriebenen Methode wird 1,2 g (0,014 mol) tert.-Butylisonitril eingesetzt. Man erhält 157,8 g (0,97 mol; 97 %) gelbes 2-Ethylhexansäurechlorid.

### Beispiel 3

Nach der in Beispiel 1 beschriebenen Methode wird 1,39 g (0,01 mol) 1,1,3,3-Tetramethylbutylisonitril eingesetzt. Man erhält 159,4 g (0,98 mol; 98 %) gelbes 2-Ethylhexansäurechlorid.

### Beispiel 4

Nach der in Beispiel 1 beschriebenen Methode wird 1,15 g (0,01 mol) N,N,N',N'-Tetramethylguanidin eingesetzt. Man erhält 160,5 g (0,986 mol; 98,6 %) gelbes 2-Ethylhexansäurechlorid, das neben Spuren des Katalysators nur noch als Verunreinigung weniger als 0,1 % des entsprechenden Carbonsäureanhydrids enthält.

### Beispiel 5

Nach der in Beispiel 1 beschriebenen Methode wird 1,35 g (0,007 mol) N-Phenyl-N',N',N'',N''-Tetramethylguanidin eingesetzt. Man erhält 160,1 g (0,984 mol; 98,4 %) gelbes 2-Ethylhexansäurechlorid, das neben Spuren des Katalysators nur noch als Verunreinigung weniger als 0,1 % des entsprechenden Carbonsäureanhydrids enthält.

## Patentansprüche

1. Verfahren zur Herstellung von Säurehalogeniden der allgemeinen Formel I 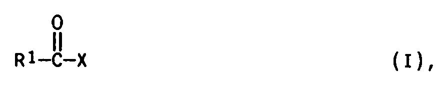 in der
R¹ C₃- bis C₈-Cycloalkyl, C₄- bis C₂₀-Cycloalkyl-alkyl oder gegebenenfalls durch C₂- bis C₄-Acyl und/oder C₂- bis C₄-Acyloxy ein- bis dreifach substituiertes C₁- bis C₃₀-Alkyl, C₂- bis C₂₀-Alkenyl, C₂- bis C₂₀-Alkinyl oder gegebenenfalls durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein- bis dreifach substituiertes Aryl, C₇- bis C₂₀-Aralkyl, Heterocycloalkyl oder C₃- bis C₂₀-Heterocycloalkyl-alkyl und
X Chlor oder Brom
bedeuten, durch Umsetzung von Carbonsäuren der allgemeinen Formel II 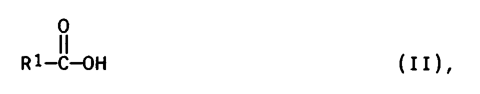 in der R¹ die oben genannten Bedeutungen hat, oder deren Anhydriden mit einem anorganischen Säurehalogenid der allgemeinen Formel III
O = AX₂ (III),
in der
A Kohlenstoff oder Schwefel bedeutet und X die oben genannten Bedeutungen hat, dadurch gekennzeichnet, daß man die Umsetzung bei Temperaturen von 40 bis 180°C in Gegenwart einer ungesättigten Stickstoffverbindung durchführt.

2. Verfahren zur Herstellung von Säurehalogeniden der allgemeinen Formel I zur Herstellung von Säurehalogeniden nach Anspruch 1, dadurch gekennzeichnet, daß man als ungesättigte Stickstoffverbindungen Guanidinderivate der allgemeinen Formel IV 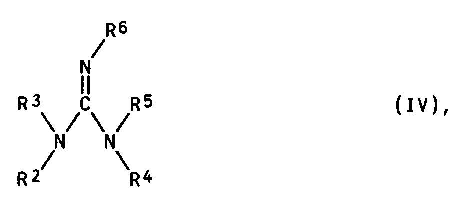 in der
R²,R³,R⁴,R⁵,R⁶ Wasserstoff, C₃- bis C₈-Cycloalkyl, C₄- bis C₂₀-Cycloalkyl-alkyl oder gegebenenfalls durch C₂- bis C₄-Acyl und/oder C₂- bis C₄-Acyloxy ein- bis dreifach substituiertes C₁- bis C₃₀-Alkyl, C₂- bis C₂₀-Alkenyl, C₂- bis C₂₀-Alkinyl oder gegebenenfalls durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein- bis dreifach substituiertes Aryl, C₇- bis C₂₀-Aralkyl, Heterocycloalkyl oder C₃- bis C₂₀-Heterocycloalkyl-alkyl
bedeuten, oder substituierte Isonitrile der allgemeinen Formel V
R⁷-N≡C (V),
in der
R⁷ C₃- bis C₈-Cycloalkyl, C₄- bis C₂₀-Cycloalkyl-alkyl oder gegebenenfalls durch C₂- bis C₄-Acyl und/oder C₂- bis C₄-Acyloxy ein- bis dreifach substituiertes C₁- bis C₃₀-Alkyl, C₂- bis C₂₀-Alkenyl, C₂- bis C₂₀-Alkinyl oder gegebenenfalls durch C₁- bis C₄-Alkyl, C₁- bis C₄-Alkoxy, Halogen, Nitro, Cyano, C₂- bis C₈-Dialkylamino, C₂- bis C₄-Acyl und/oder C₂- bis C₄-Acyloxy ein- bis dreifach substituiertes Aryl, C₇- bis C₂₀-Aralkyl, Heterocycloalkyl oder C₃- bis C₂₀-Heterocycloalkyl-alkyl
bedeutet verwendet.

3. Verfahren zur Herstellung von Säurehalogeniden der allgemeinen Formel I nach Anspruch 1, dadurch gekennzeichnet, daß 1,0 bis 1,5 Mol anorganisches Säurechlorid III pro Äquivalent zu bildender Carbonsäurehalogenidgruppe eingesetzt werden.

4. Verfahren zur Herstellung von Säurehalogeniden der allgemeinen Formel I nach Anspruch 1, dadurch gekennzeichnet, daß man als anorganisches Säurehalogenid III Phosgen oder Thionylchlorid einsetzt.

5. Verfahren zur Herstellung von Säurehalogeniden der allgemeinen Formel I nach Anspruch 1, dadurch gekennzeichnet, daß man die ungesättigte Stickstoffverbindung IV und/oder V in Menge von 0,001 bis 10 Mol %, verwendet.
